# EUROPEAN PATENT APPLICATION

(11) **EP 1 300 161 A1**
(43) Date of publication of application: **09.04.2003**
(21) Application number: 01123973.8
(22) Date of filing: 08.10.2001
(51) Int. Cl.: A61K 48/00, C07H 21/00, C12N 15/87

(54) **Cationic lipids suitable for gene transfer**

(71) Applicant: rubitec Gesellschaft für Innovation und Technologie der Ruhr-Universität Bochum mbH, 44801 Bochum (DE); Ruhr-Universität Bochum, 44780 Bochum (DE); Albert-Ludwigs-Universität Freiburg, 79104 Freiburg (DE); KTB Tumorforschungsgesellschaft mbH, 79011 Freiburg (DE)
(72) Inventor: Von Kiedrowski, Günter, Prof.Dr., 44797 Bochum (DE); Lenssen, Karl Christian, 42555 Velbert (DE); Jantscheff, Peter, Dr., 79211 Denzlingen (DE); Massing, Ulrich, Dr., 79249 Merzhausen (DE); Burger, Angelika Maria, P.D.Dr., 79104 Freiburg (DE)
(74) Representative: Helbing, Jörg, Dr. Dipl.-Chem.

(57) **Abstract**

The present invention relates to compositions for transferring biologically active molecules into cells comprising particular cationic lipids such as di- and trialkylamino alkanols, preferably *N,N,N*-trialkylamino-2,3-propanediols, and the use of said cationic lipids for preparing gene transfer agents.

## Description

The present invention relates to compositions for transferring biologically active molecules into cells comprising particular cationic lipids such as di- and trialkylamino alkanols, preferably *N,N,N*-trialkylamino-2,3-propanediols, and the use of said cationic lipids for preparing gene transfer agents.

### Background of the Invention

Gene therapy is a promising strategy for treating acquired or inherited genetic diseases such as cancer or cystic fibrosis. The fundamental hurdle in the transfer of *gene therapy* from the experimental stage to clinical practice is the development of vectors to deliver genetic material (therapeutic genes) to the appropriate cells in a specific, efficient, and safe manner. This problem of "drug delivery," where the drug is a gene, is particularly challenging for genes which are large and complex and which require targeting to the nuclei of cells. Most of the vectors currently in use for clinical gene therapy trials are based on attenuated or modified versions of viruses. Although efficient, there are serious safety problems associated with viral vectors including possible activation of the patients immune system, risk of infection with traces of the wild type virus or insertion mutagenesis. These risks were dramatically underscored by the death of an 18-year old man in an adenovirus based gene therapy study in 1999.

A promising alternative is gene transfer mediated by cationic lipids (lipofection): Cationic lipids have the advantage of directly interacting with the polyanion DNA, thereby forming lipid-DNA complexes (Massing, U. Lipid Technology 13(3):57-60 (2001)). These mostly positively charged complexes, or lipoplexes, are supposed to bind to and be endocytosed by (negatively charged) cells. The transferred genetic material (DNA, RNA, or oligonucleotides) then has to escape lysosomal degradation and to enter the nucleus to reach its target. Lipofection bears advantages over viral transfection: There is no restriction on the size of the therapeutic gene and no risk of immunogenicity or infection. Additionally, cationic lipids can be manufactured in large quantities with relatively little effort.

However, in contrast to viral gene transfer, the efficiency of lipofection is still poor and the mechanism of lipofection is only partly understood.

Such cationic lipids are known in the art. E.g. WO 01/57064 and Fichert, T. et al., Bioorg. Med. Chem. Letters, 10:787-791 (2000) disclose a cationic amphiphile consisting of a cholesterol or diacylglycerol moiety serving as the lipid anchor, a spacer group and a (poly)amine based cationic head group, which forms part of a lipid mixture or a liposome. Said lipid mixture or liposome may additionally contain a helper lipid. However, the synthesis of the cationic lipids described here is quite cumbersome, it requires protecting groups, and is carried out in solution phase. It is desirable to have alternative lipid anchors at hand which are easier to synthesize from readily available starting materials e.g. by means of solid phase synthesis not involving the use of protecting groups. The applicability of solid phase synthesis would also facilitate the implementation of a rapid screening process in order to find those lipids which offer optimum efficiency for the transfer of a biologically active molecule into a cell.

DE 199 25 143 describes as an alternative liposomal vector complexes (comprising a nucleic acid sequence of any length, a cationic carrier, and lipids and phospholipids forming a liposome), a process for their preparation and their use in the preparation of a drug for prophylactics or therapy of a disease. Thus, this alternative is limited to liposomes where the cationic carrier is present as an additional component. The latter has to be purchased or synthesized and requires an additional step in the preparation of the liposomal vector complexes. This also renders a screening process for finding the lipid mixture with optimum properties more laborious. By the use of a cationic lipid this cationic carrier could be omitted.

DOPE (N,N-dioleoyl phophatidylehtanolamine) alone or in a mixture with DOTAP (N-(1(2,-dioleyoyl)propyl)-N,N,N-trimethylammonium chloride) and other helper lipids are known to form lipoplexes suitable for gene delivery (Hirsch-Lerner, D. and Barenholz, Y., Biochim. Biophys. Acta, 1461:47-57 (1999) and 1370:17-30 (1998); De Olivera, M.C. et al., Biochim. Biophys. Acta 1372:301-310 (1998)).

Finally, DOTAP analoga and their use in Lipofection is described in Regelin, A.E. et al., BBA 1464:151-164 (2000) and in Massing, U. et al., Chem. Phys. Lipids 105:189-191(2000).

On the other hand, JP-A-01233264 discloses a quarternary ammonium salt compound of the formula 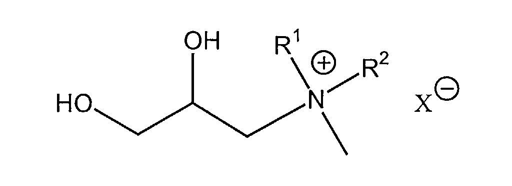 wherein R¹ and R² are saturated C₈₋₁₈ alkyl groups and X is halogen which can be utilized as germicide, e.g., for washing tableware and disinfecting livestock barns. JP-A-02223515 utilizes the above quaternary ammonium compounds (where R¹ and R² are saturated C₈₋₁₄ alkyl groups and X is halogen) as antifungal agent. Finally, JP-A-02225490 discloses quaternary ammonium salts of glucose or methyl glycoside (such as (6-deoxy-D-glucopyran-6-yl)didecylmethylammonium chloride) which are useful as a germicide, e.g., for dish-washing and cattle stall sterilization.

### Description of the Invention

In a previous study with analogs of the transfection lipid DOTAP which differed in their non-polar hydrocarbon chains, it was shown that the transfection efficiency strongly depends on the biophysical properties of the transfection lipids, of the resulting liposomes and at the lipoplexes (Regelin, A.E. et al., BBA 1464:151-164 (2000); Massing, U. et al., Chem. Phys. Lipids 105:189-191(2000)). Minimal alterations of biophysical properties by using lipids with different hydrocarbon chains or by mixing the lipid with different helper lipids could completely allow or prevent transfection. Prediction of transfection properties from a lipids structure is, however, not possible.

It was now found that particular cationic lipids of formula (I) shown below possess a transfection behavior superior to that of DOTAP. These cationic lipids are readily accessible by a solid phase synthesis and may be utilized to develop optimized lipofection protocols for different cell types. The invention thus provides:
(1) a composition for transferring biologically active molecules into cells and/or tissues comprising
   (a) a cationic lipid of formula (I) 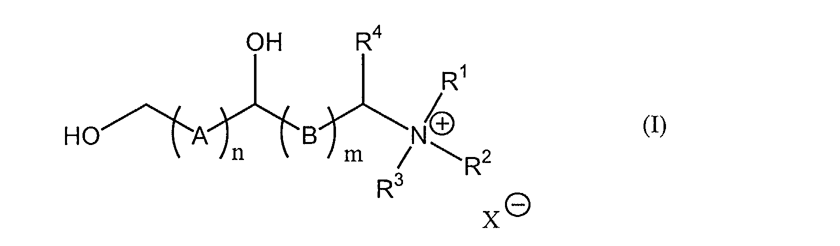 wherein
      - R¹, R² and R³: are the same or different and are selected from hydrogen and an alkyl group which may be substituted, provided that at least one of R¹, R² and R³ is an alkyl group which may be substituted and that the total number of carbon atoms within R¹, R² and R³ is at least 18,
      - R⁴: is hydrogen or a lower alkyl residue having about 1 to 6 carbon atoms which may be substituted,
      - A and B: are the same or different and are selected from-C(=O)- -C(OH)R⁵- and -CR⁵R⁶- (wherein R⁵ and R⁶ are hydrogen or a lower alkyl residue having about 1 to 6 carbon atoms which may be substituted),
      - m and n: are independently an integer of 0 to 3, and
      - X: is a biologically and/or pharmaceutically acceptable counter ion, or derivatives of the lipid of formula (I); and
   (b) at least one biologically active molecule;
(2) in an preferred embodiment of (1) above the cationic lipid has the formula (Ia): 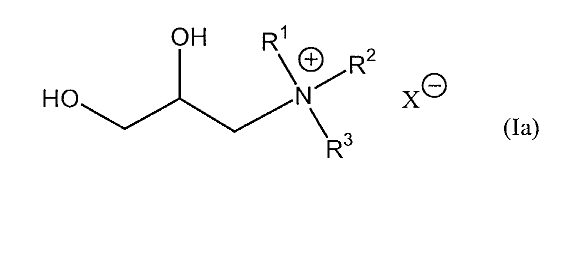 wherein R¹, R² and X are as defined in (1) above and R³ is hydrogen, a methyl group or a hydroxyethyl group;
(3) the use of a cationic lipid of formula (I) as defined in (1) and (2) above
   (i) as constituent of a transfection agent for transferring a biologically active molecule into cells and/or tissues *in vitro* or
   (ii) for preparing an agent for transferring a biologically active molecule into cells and/or tissues *in vivo* including, but not limited to, an agent for gene therapy;
(4) a cationic lipid of the formula (I) as defined in (1) and (2) above, provided that a compound is excluded where m and n are 0, R¹ and R² are saturated, linear and unsubstituted alkyl groups having 8 to 18 carbon atoms, R³ is methyl, R⁴ is hydrogen and X is a halide;
(5) a process for the manufacture of the cationic lipid as defined in (1) and (2) above which comprises
   (a) reacting a compound of the formula (II) 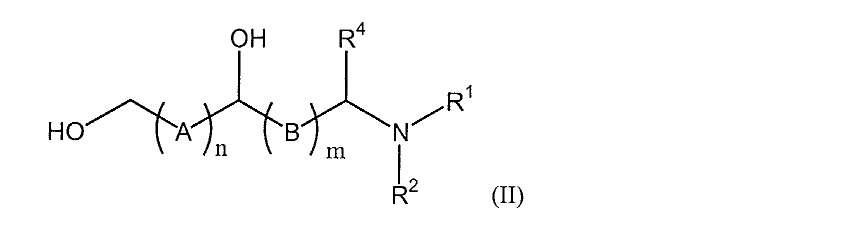 with a compound of the formula (III)

      R³-Y (III)
   wherein all variables are as defined in claims 1 to 5 and Y is a leaving group; and
(6) a method for transferring biologically active molecules into cells and/or into tissues or for gene therapy which method comprises contacting the cells or tissues, or the subject in need of the gene therapy with a composition comprising a cationic lipid of formula (I) as defined in (1) and (2) above.

### Figures

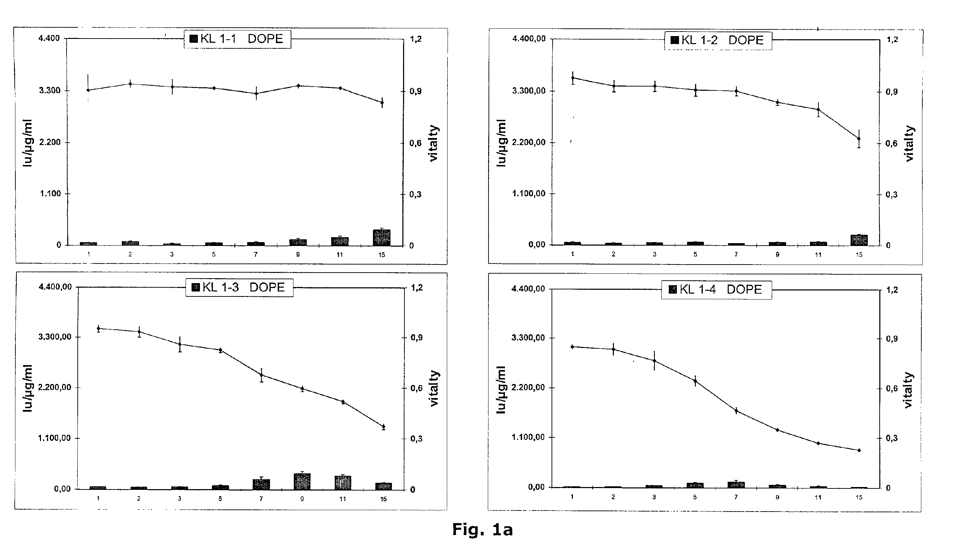

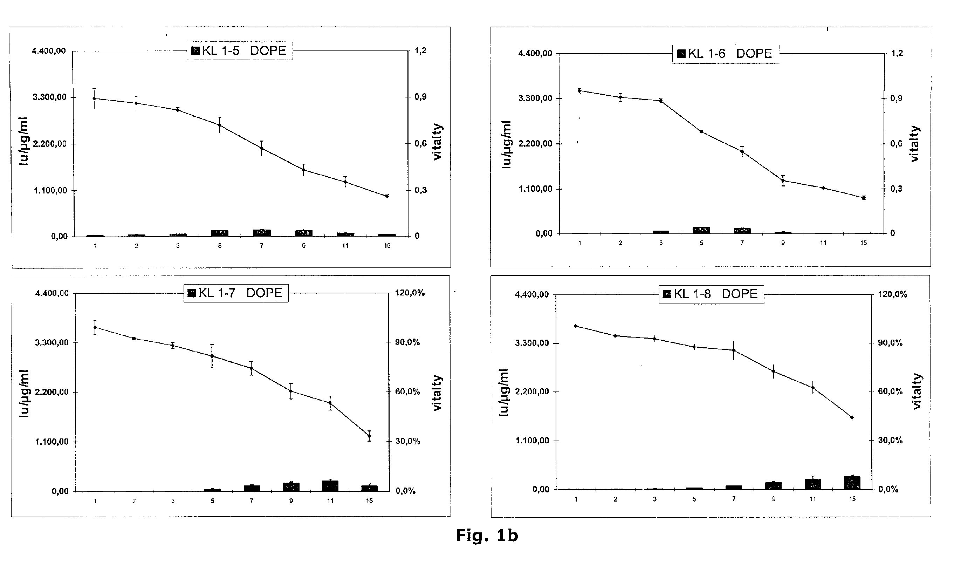

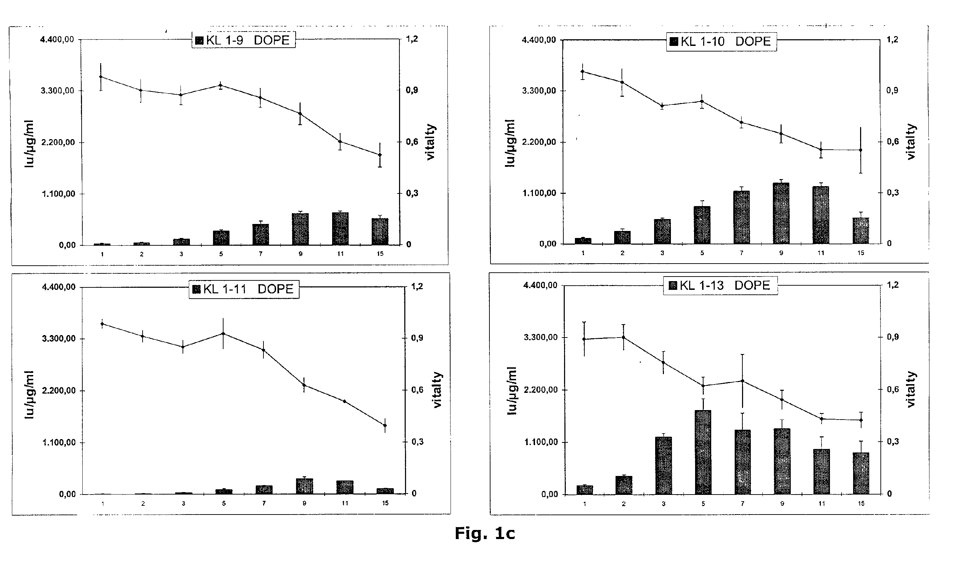

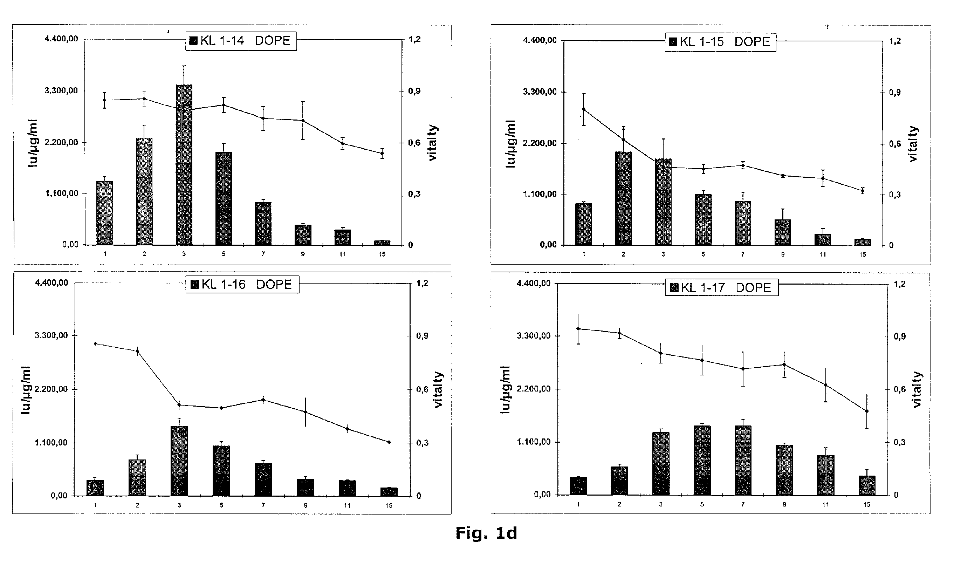

Figure 1: Lipofection results (lipofection profiles) of lipoplexes from the R-configurated KL-1 - KL-17 in a mixture with equimolar amounts of DOPE (counter ion: chloride) and the pCMVluc-plasmid. Each bar represents the mean (± S.D.) of 3 wells of a 96-well microtiter plate.

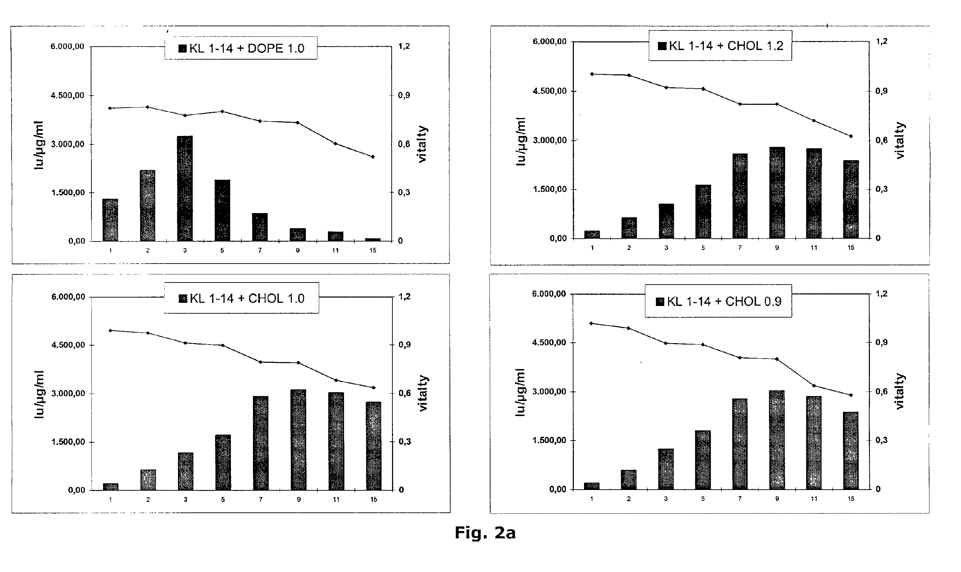

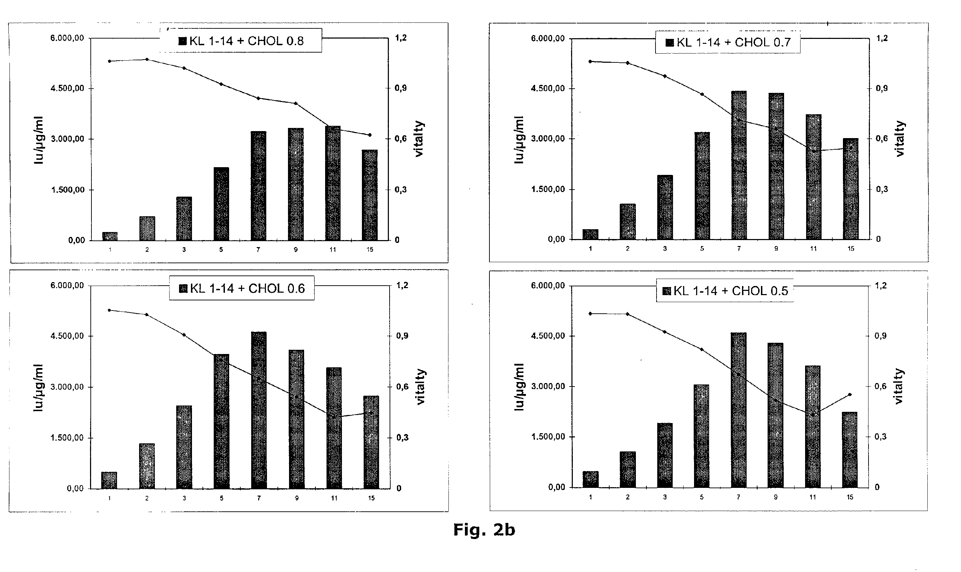

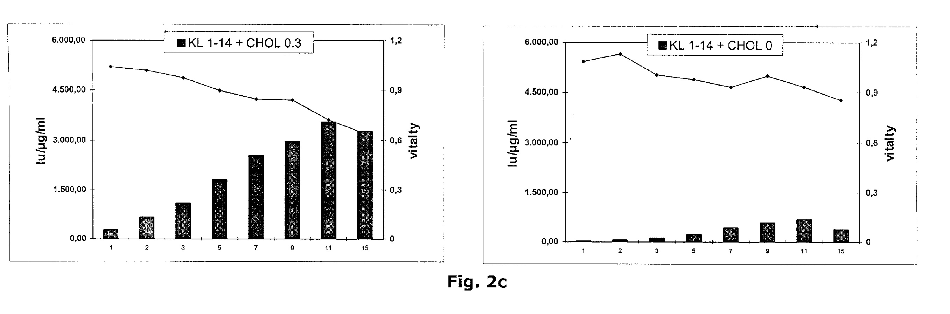

Figure 2: Lipofection results (lipofection profiles) of lipoplexes from the R-configurated KL-14 (contains two C14 hydrocarbon chains) in a mixture with cholesterol in different cholesterol/cationic lipid-ratios from 0.3 - 1.2 (counter ion: chloride) and the pCMVluc-plasmid. Each bar represents the mean (± S.D.) of 3 wells of a 96-well microtiter plate.

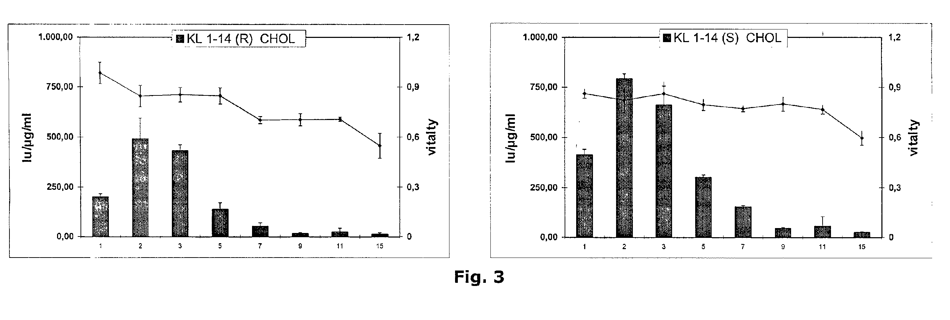

Figure 3: Lipofection results (lipofection profiles) of lipoplexes from the R-configurated (A) and S-configurated KL-14 (B) in a mixture with cholesterol in a cholesterol/KL-14-ratio of 0.7 (counter ion: chloride) and the pCMVluc-plasmid. Each bar represents the mean (± S.D.) of 3 wells of a 96-well microtiter plate.

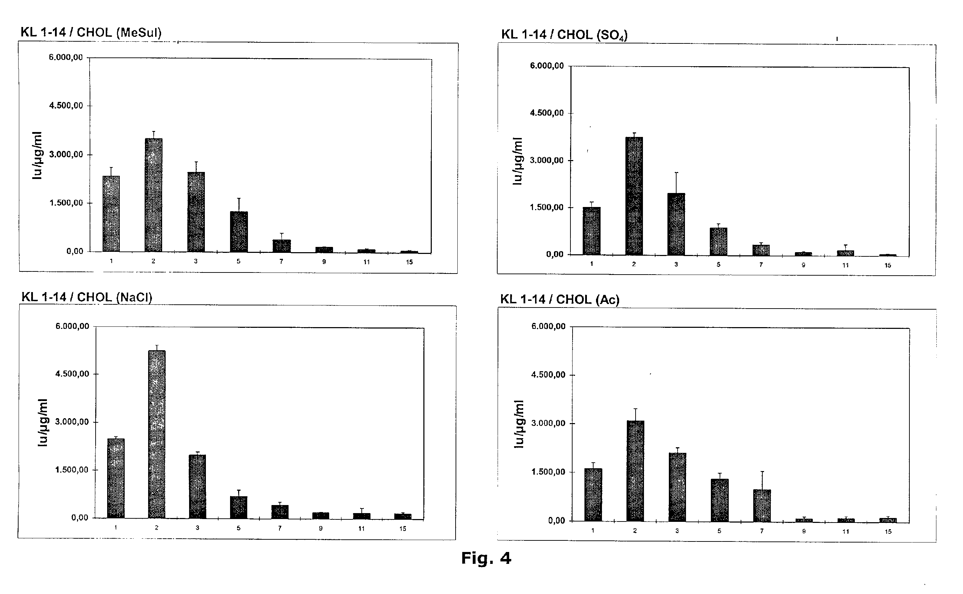

Figure 4: Lipofection results (lipofection profiles) of lipoplexes from the racemic KL-14 in a mixture with cholesterol in a cholesterol/KL-14-ratio of 0.7 with different counter ions: (A: methyl sulfate, B: sulfate, C: chloride, D: acetate) and the pCMVluc-plasmid. Each bar represents the mean (± S.D.) of 3 wells of a 96-well microtiter plate.

### Detailed Description of the Invention

The complex between cationic lipid (a) and biologically active molecule in the composition of embodiment (1) of the invention is hereinafter shortly referred to as "lipoplex".

In the composition for transferring biologically active molecules into cells of embodiment (1) of the inventionit is preferred that the compound of formula (I) has at least two alkyl groups which may be substituted. Moreover, it is preferred that R¹ and R² are linear or branched chain, saturated or unsaturated alkyl residues having at least about 8, preferably 10 to 26 carbon atoms which may be substituted. Still more preferred is that R¹ and R² are linear alkyl residues having 10 to 22 carbon atoms, most preferable 12 to 20 carbon atoms. It is particularly preferred to utilize naturally occurring C₁₆, C₁₈, C₂₀ fatty acid derivatives, e.g., residues derived from oleic acid, linoleic acid, linolenic acid, Cis-11-eicosenic acid, arachidonic acid and erucic acid, or the aldehydes or alcohols corresponding thereto. Moreover, it is preferred that R³ is hydrogen or a saturated lower alkyl residue having about 1 to 6, preferably 1 to 3 carbon atoms which may be substituted. Still more preferred is that R³ is a methyl group or a hydroxy-lower-alkyl group. More preferred it is that R⁴ is hydrogen, a lower alkyl group or a hydroxy-lower-alkyl group.

The above lower alkyl groups having about 1 to 6 carbon atoms within the definition of R³ and R⁴ include linear and branched, saturated and unsaturated residues such as methyl, ethyl, propyl, isopropyl, n-butyl, sec-butyl, tert-butyl, n-pentyl, n-hexyl, alkyl and the like.

Suitable substituents on the alkyl group and lower alkyl group include, but are not limited to, the following groups: hydroxy, oxy, halogen (such as fluorine, chlorine, bromine etc.), lower alkyl (a linear or branched alkyl group having about 1 to 6 carbon atoms as defined hereinbefore), acyl (such as formyl, acetyl, propionyl, butyryl, etc.), acyloxy (including, but not limited to, formyloxy, acetyloxy, propionyloxy, seryloxy, glycyloxy, alanyloxy etc.), mercapto, lower alkyl thio (such as methylthio, ethylthio, propylthio, isopropylthio, butylthio, etc.) and the like. The number of such substituents on the alkyl residues of R¹, R², R³ and R⁴ is 1-5, preferably 1-3.

As biologically and/or pharmaceutically acceptable counterion any mono-, di- or polyvalent counterion which does not adversely affect the transfer of the biologically active molecule into the cell or tissue can be utilized. Preferred counterions include, but are not limited to, carboxylates (such as acetate, propionate, etc.), fluorinated carboxylate (such as monofluoroacetate, difluoroacetate and trifluoroacetate, perfluoropropionate and the like), sulfate, alkylsulfates (such as methylsulfate, ethylsulfate, propylsulfate and the like), fluorinated alkylsulfates (the alkylsulfates mentioned hereinbefore containing at least one fluor atom), arylsulfates (including alkylaryl sulfates such as benzylsulfate, toluylsulfate and the like), fluorinated arylsulfates (the arylsulfates mentioned hereinbefore containing at least one fluor atom) and halides (such as chloride and bromide).

In the compound of formula (I), in case m or n are 2 or 3, the groups A and B, respectively, may be identical or may vary among its possible meanings. Derivatives of the lipid of formula (I) include, but are not limited to, estrification products at the primary or secondary hydroxy group or amino acids such as serine, glycine, alanine, etc.); oxidation at the secondary hydroxy group to yield a hydroxy ketone, which may further be derivatized, e.g. by reductive animation with R⁷-NH₃ along the scheme set forth below. 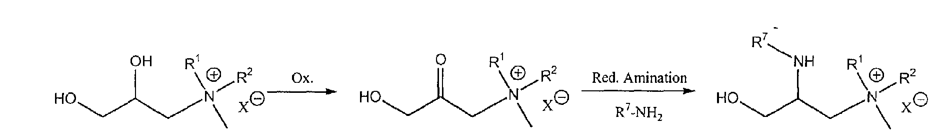 wherein R⁷ may have the same meaning as R³.

Embodiment (2) of the invention pertains to compounds of formula (I) where n and m are 0, R³ is hydrogen, a methyl group or a hydroxyethyl group and R⁴ is hydrogen, i.e., the compound possesses the formula (Ia) 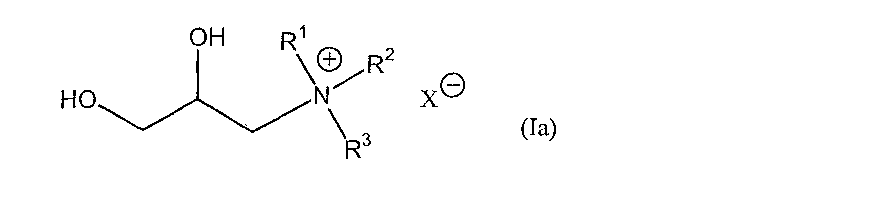 wherein all variables are as defined above. In said embodiment (2) it is preferred that R¹ and R² are saturated or unsaturated, linear C12, C14, C16, C18, C20 or C22 alkyl residues, R³ is a methyl group, and X is halide, acetate, methylsulfate or sulfate. Most preferable X is chloride. In compounds of the formula (Ia), the configuration of C(2) is racemic or has either R or S configuration.

In accordance with embodiment (1) and (2) of the invention the biologically active molecules include, but are not limited to, nucleic acid constructs, DNA, RNA, synthetic polynucleotides, antisense polynucleotides, missense polynucleotides, nonsense polynucleotides, ribozymes, proteins, peptides, small molecular weight drugs, antibiotics and hormones.

The composition of embodiments (1) and (2) of the present invention may additionally comprise a helper lipid (c) which differs from the cationic lipid of formula (I) or (Ia), or a mixture of such helper lipids. The helper lipid(s) is/are preferably selected from phosphatidylcholine, lyso-phospatidylcholine, phosphatidylethanolamine and lyso-phosphatidylethanolamine and the like (having saturated or unsatured carbon chains of variable length); sphingomyelin, ceramide and the like; membrane forming amphiphiles such as block polymers and the like; alkylester, -ether, -amides of sugars, dioles, polyoles, amines, amino acids, peptides and the like; cationic or lipophilized polymers such as DEAE-dextran, histones, polyethylene imine and the like; cholesterol; phosphatidylglyceroles, phosphatidylserines, phosphatidylinositoles and the like; helper agents modulating uptake of lipoplexes by macrophages and other cells such as gangliosides, phosphatidylpolyglycideroles, PEG-phosphatidylethanolamines, proteins (including IgG and Fc-fragments) and the like. Most preferable the helper lipid is dioleoylphospatidylethanolamine (DOPE) or cholesterol.

In accordance with the present invention the molar ratio of (c)/(a) is from 0.01 to 20, preferably 0.1 to 5, and more preferably is from 0.3 to 1.2 in the case of DOPE and from 0.3 to 1.0, most preferably from 0.5 to 0.7, in the case of cholesterol.

In a further preferred embodiment of the invention the composition of embodiments (1) and (2) the composition is a composition for gene therapy, i.e., is suitable for transferring a biologically active molecule into a mammalian, preferably human cells.

The present invention also provides the use of a cationic lipid of formula (I) or (Ia) as defined hereinbefore as a transfection agent for transferring a biologically active molecule into cells and/or tissues (i.e., in an *ex vivo* application), or for preparing an agent for gene therapy (i.e., *in vivo* application) for mammals including humans. Besides the biologically active molecule and the cationic lipid, the agent may further comprise a helper lipid as defined hereinbefore.

The present invention also provides a cationic lipid of the formula (I) as defined hereingefore, provided that a compound is excluded where m and n are 0, R¹ and R² are saturated linear and unsubstituted alkyl groups having 8 to 18 carbon atoms, R³ is methyl, R⁴ is hydrogen and X is a halide (the disclaimer being directed to exclude the compounds disclosed in JP-A-01233264 and JP-A-02225490). Moreover, the invention provides a process for the manufacture of said the cationic lipids, which comprises
(a) reacting a compound of the formula (II) 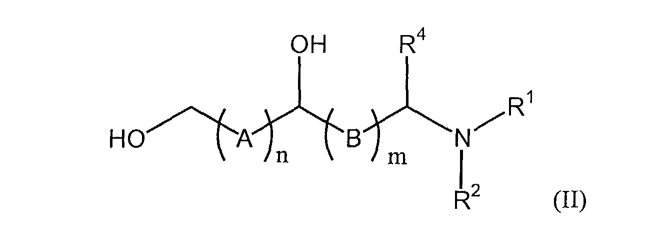 with a compound of the formula (III)

   R³-Y (III)
wherein all variables are as defined hereinbefore and Y is a leaving group.

A complete synthesis strategy utilizing a combinatorial solid phase synthesis for cationic (transfection) lipids of the invention is shown below. The strategy is based on the utilization of 4-methoxytrityl chloride resin. In order to gain access to a large number of compounds only commercially available building blocks were used. Protective groups were omitted if possible. The structure is based on 3-methylamino-1,2-dihydroxy-propane as the polar, cationic lipid part. As non-polar lipid part, different hydrocarbon chains are bound to the amino group of this scaffold. The amino group is further methylated to get a constantly cationic charged lipid. The synthesis allows to synthesize the lipids of the invention in different configuration, and with different counter ions.

The general synthetic route for the preparation of the cationic lipid of the present invention is exemplified for the lipid of embodiment (2) outlined below.

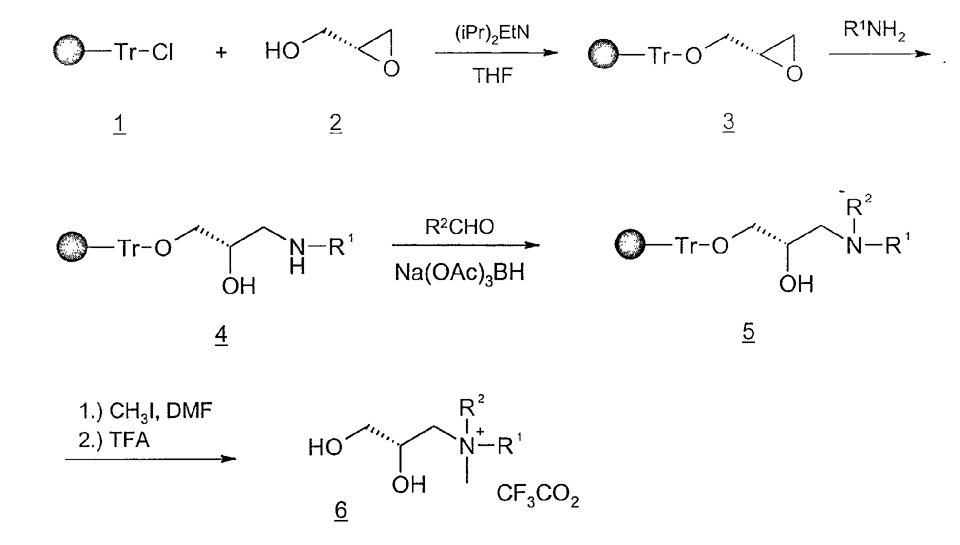

The synthesis started with the immobilization of (*R*)-2,3-epoxy-1-propanol **2** on the 4-methoxytrityl chloride resin **1.** Reaction of the epoxide **3** with a long-chained amine yielded the polymer-bound secondary amine **4,** with is converted to the tertiary amine **5** by reductive amination. Quaternization of the tertiary amine by methyl iodide and cleavage from the solid phase gave the cationic lipid **6**, which was further purified by prep. HPLC. Each step of the synthesis could be monitored by means of HR-MAS-NMR (HRMAS = high resolution magic angle spinning). Lipids with different alkyl chain length, different chirality and different counter ions have been prepared by this synthetic route (see table 1). Larger amounts of racemic lipid **6** were synthesized by alkylation of 3-methylamino-1,2-propandiol with 1-Bromotetradecane.

The lipids **6** which were prepared by this route are summerized in table 1 shown below. This library of N,N-dialkyl-N-methyl-amino-2,3-propandiols with alkyl groups of different length were synthesized in R- or S-configuration or as racemat. As counter ions, chloride, sulfate, methylsulfate and acetate were used, respectively.

**Table 1**

| name | R¹ | R² | Name | R¹ | R² |
|---|---|---|---|---|---|
| KL-1 | C₁₀ | C₈ | KL-9 | C₁₆ | C₁₀ |
| KL-2 | C₁₂ | C₈ | KL-10 | C₁₈ | C₁₀ |
| KL-3 | C₁₄ | C₈ | KL-11 | C₁₂ | C₁₂ |
| KL-4 | C₁₆ | C₈ | KL-13 | C₁₈ | C₁₂ |
| KL-5 | C₁₈ | C₈ | KL-14 | C₁₄ | C₁₄ |
| KL-6 | C₁₀ | C₁₀ | KL-15 | C₁₄ | C₁₆ |
| KL-7 | C₁₀ | C₁₂ | KL-16 | C₁₈ | C₁₄ |
| KL-8 | C₁₀ | C₁₄ | KL-17 | C₁₆ | C₁₂ |

The synthesis of compounds where m and n are ≠ 0 may proceed according to a similar route shown below.

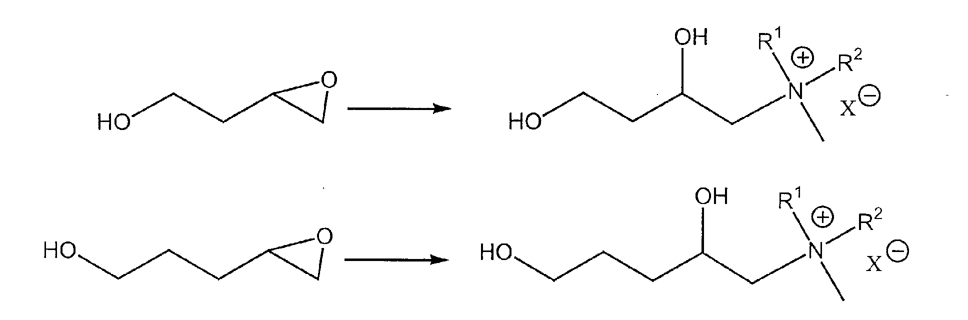

Screening / Lipofection studies: For a broad and reproducible testing the transfection properties of systematically varied new cationic lipids, we recently have developed an fully automated high throughput system (HTS) (Regelin, A. E. et al., J. Biomol. Screening (2001)). This system, which comprises the entire lipofection process from liposome formation to reporter gene assay, allows to identify candidate cationic lipids and to develop suitable lipofection reagents and protocols based on those lipids. Automation of this process was necessary, because manually testing of various cationic lipids or lipid mixtures is extremely labor intensive and high reproducibility can not be guarantied.

### (i) Screening the combinatorial lipid library for lipofection properties:

Goal of this first step was to find the cationic lipid with the best hydrocarbon chain composition for the development of suitable lipofection reagents and protocols. All lipids with different hydrocarbon chains (lipids 1-16, table 1) which are R-configurated and have chloride as counter ion were tested using a standard protocol: From each lipid, eight different lipoplexes containing equimolar amounts of the helper lipid DOPE were formed using different DNA:lipid-charge ratios from 1:1 - 1:15, resulting in a lipofection profile for each lipid (fig. 1). The COS-7 cell-line was used for this first lipofection study because COS-7 is easy to transfect and differences between the lipids are easy to identify. For comparison, the transfection efficiency of the well know cationic lipid DOTAP at its best lipid/DNA charge ration of 2.5 was also determined in each run.

Compared to DOTAP, all new lipids showed better transfection efficiencies ranging from about 220 to 3400 rlu/µg/ml protein, which corresponds to 250 - 6.000 % of the DOTAP values. The transfection efficiencies strongly depend on the hydrocarbon chains of the lipids. In general, increasing overall length of the hydrocarbon chains resulted in higher transfection efficiencies: An overall length of at least 28 CH-units seems to be necessary for transfection efficiencies 10fold higher than those of DOTAP. But also the combination of the hydrocarbon chains seems important. Comparing the lipids KL-10, 14 and 17 which all have a total of 28 CH-units, the lipid KL-14 bearing two C-14 hydrocarbon chains was the most effective.

The transfection profiles of the most effective lipids of this group are similar, showing a peak (highest transfection efficiency) at Lipid/DNA ratios from 2 - 5. For the most effective lipids, the vitality of the cells at maximum transfection efficiency usually decreased to roughly 50%. An exception is the lipid KL-14, which shows the highest transfection efficiency as well as only a minor toxicity of roughly 70% vitality. We chose lipid KL-14 for further development of a versatile transfection reagent.

### (ii) Influence of helper lipids on transfection effeciencies

The previous screening experiments were performed with lipoplexes containing equimolar amounts of the helper lipid DOPE. Here the influence of different ratios of the helper lipids DOPE and Chol on transfection efficiency of KL-14 were tested. Furthermore, the transfection behavior of KL-14 without any helper lipid was tested.

Transfection efficiency of KL-14 without helper lipids was very low and reached only 70% of the transfection efficiency which was found for the standard lipid DOTAP. Independent of the amount of DOPE incorporated in the lipoplexes (ratio of DOPE/KL1-14: 0.3, 0.5, 0.6, 0.7, 0.8, 0.8, 1.0 and 1.2) the transfection efficiency was very high as previously described (see above). The transfection efficiency and the transfection profiles were very similar for all DOPE/KL-14-ratios.

Using Cholesterol (Chol) as helper lipid for KL-14, the transfection efficiency were no longer similar for different Chol/KL-14-ratios (fig. 2), but always higher than for the mixtures of KL-14 with DOPE. Highest transfection efficiency were found for Chol/KL1-14-ratios of 0.5-0.7. Higher or lower rations led to decreased transfection efficiency. Toxicity of all mixtures were similar and moderate.

### (iii) Influence of the configuration, methylation and the counter ion of KL-14 on transfection efficiency

We compared the transfection efficiency and toxicity of KL-14 synthesized in the R- and S-configuration (with Chol as helper lipid (see above)). The results for both lipid were statistically similar. Nevertheless, there was a trend toward higher transfection efficiency and lower toxicity for the S-configurated KL-14. For further experiments, KL-14 were synthesized as racemat.

Methylation of KL-14 was an important prerequisite for its transfection properties. An KL-14-analog which was not methylated did not transfect at all. It could be assumed that the non-methylated KL-14 was not sufficiently protonated at physiological pH so that the formation of a bilayer structure from these lipids could not occur. As previously shown for DOTAP-analogs, formation of bilayer is an prerequisite for a cationic lipid to be a transfection lipid.

The influence of four different counter ions on transfection behavior of KL-14 was tested. As shown in fig. 4, chloride as counter ion resulted in highest transfection efficiency. Using methylsulfate or sulfate as counter ion, transfection efficiency was reduced to 70-75% of the values found for chloride. Acetat as counter ion led to the lowest transfection efficiency, which was only 57 % of the chloride values.

### (iv) Transfection properties toward different cell lines

For testing the transfection properties of KL-14 toward the mamma carcinoma cell lines MDA-MB-468 and MCF-7, the polarized cell line MDCK-C7 and the primary dendritic cells, KL-14 was used in its racemic form with chloride as counter ion and as a mixture with 70 mol-% cholesterol. In comparison with COS-7-cells (see above), the transfection efficiencies were generally lower: For the mamma carcinoma cell lines MDA-MB-468 and MCF-7, transfection efficiencies were reduced by a factor of about 12, the MDCK-C7-cells by a factor of about 100 and the dendritic cell by a factor of about 600. Nevertheless, the transfection efficiencies found for KL-14 was generally higher than for DOTAP. For the mammacarcinoma cell lines, transfection efficiencies with KL-14 were 8 times higher than for DOTAP. For MDCK-C7 and dendritic cells, the increase was 1.5-2fold.

However, we also tested KI-14 as equimolar mixture with DOPE for its transfection efficiencies toward the four cell types. For the mamma carcinoma cell lines, it could be confirmed that transfection efficiencies were lower with the DOPE-formulation.

Improving and easing the process of transfection is the key for establishing gene therapy in clinical practice. Today, compared to viral transfection techniques, lipofection is less effective, but it has strong advantages concerning safety aspects, versatility and also the manufacturing aspects. To become a method routinely used, lipofection has to be improved.

This application describes the first synthesis-screening approach for the identification and optimization of new cationic lipids for gene transfer in various cell lines. For the first time, combinatorial solid phase chemistry was used to synthesize a library of new cationic lipids, differing in their non-polar lipid part, their configuration and their counter ions.

From the combinatorial cationic lipid library, the lipid KL-14 was identified by systematic screening as a suitable lipid for the development of a new transfection reagent. Continuing systematic investigations of the transfection conditions revealed that cholesterol is a suitable helper lipid and that the best KL-14/cholesterol ratio is 0.7. Furthermore, it could be shown that the configuration of the lipids has no influence on the transfection efficiency that chloride as counter ion led to the highest transfection efficiencies.

Using this new transfection reagent, its transfection behavior toward various cells which are important for the development of new gene therapy strategies was compared. It could be demonstrated that KL-14 showed even in these cell types improved transfection efficiencies, which were 1.5-8 times higher than using the cationic lipid DOTAP.

The synthesis-screening approach presented in this application might also be useful for identification of optimal transfection reagents for therapeutic gene transfer. Further studies using other cell systems and new lipid libraries might contribute to transfer gene therapy from an experimental stage to a widely used clinical standard.

The invention is explained in more detail in connection with the following examples, which are, however, not to be construed to limit the invention.

### Examples

### I. Synthesis of the Cationic Lipids

Material and Methods: THF was distilled over sodium prior to use, CH₂Cl₂ and dimethylformamide were distilled over CaH₂. 4-methoxytrityl chloride resin was purchased from Advanced Chemtech, all other chemicals were of highest commercial purity and purchased from Sigma, Fluka or Acros. ¹H-HRMAS-NMR spectra (600 MHz) were recorded on a Bruker DRX-600 instrument in CDCl₃.

Identity of the products was verified by matrix-assisted laser desorption/ionization time-of-flight mass spectrometry (MALDI-TOF; 2,5-dihydroxybenzoic acid (DHB) as matrix) and ¹H-NMR-spectroscopy (400 MHz). Lipid-libraries were synthesized by a SYRO II parallel synthesizer. Prep. HPLC purification was performed on a Shimadzu LC-8A chromatograph, equipped with an evaporative light scattering detector (column material: Kromasil 5 NH₂; CHCl₃/MeOH 95:5).

### Experimental procedure for the synthesis of alipid containing a C₁₆ and C₁₂ hydrocarbon chain.

A. Immobilisation reaction: A solution of (*R*)-2,3-epoxy-1-propanol (65 mg) and N,N-diisopropylethylamine (135 mg) in anhydrous THF (4 ml) was added to 4-methoxytrityl chloride resin (50 mg, 0,085 mmol). After stirring the suspension for 48 h, the resin was filtered, washed several times with CH₂Cl₂/Methanol/*N,N*-Diisopropylethylamine (17:2:1), THF and CH₂Cl₂, and dried in vacuo. ¹H-HRMAS-NMR (600 MHz, CDCl₃): δ (ppm) = 2.5 (s, -CHOC*H*H), 2.7 (s, HOC*H*H-), 3.0 (s, - CH2C*H*O-), 3.3 (s, -CHOCH*H*) 3.4 (s, - HOCH*H*-), 3.7 (s, -OCH3).

Synthesis of secondary amine (4): The resin bound epoxide (3) was suspended in absolute ethanol (4 ml). Hexadecyl amine (210 mg, dodecyl-, tetradecyl- and octadecyl amine were also used) was added and the reaction mixture was heated to 65 °C for 12 h. The resin was filtered, washed several times with Ethanol, THF and CH₂CL₂, and dried in vacuo. ¹H-HRMAS-NMR (600 MHz, CDCl₃): δ (ppm) = 0.9 (s, -CH₃), 1.3 (s, -CH₂), 3.6 (s, -OCH₂-, -CHO-, -CH₂N-), 3.7 (s, -OCH₃).

Synthesis of tertiary amine (5): The intermediate resin (4) and sodiumtriacetoxy borohydride (185 mg) were suspended in anhydrous CH₂Cl₂. Dodecanal (160 mg, octanal, decanal and tetradecanal were also used) was added and the reaction mixture was stirred for 3 h. The resin was filtered, washed with methanol and CH₂Cl₂, and dried in vacuo. ¹H-HRMAS-NMR (600 MHz, CDCl₃): δ (ppm) = 0.9 (s, -CH₃), 1.3 (s, -CH₂), 3.6 (s, -OCH₂-, -CHO-, -CH₂N-), 3.7 (s, -OCH₃).

Quaternisation of tertiary amine (4): The resin-bound tertiary amine (4) was suspended in anhydrous DMF. Methyl iodide (120 mg) was added and the mixture was stirred for 12 h. After this the resin was filtered, washed with DMF and DCM, and dried in vacuo. ¹H-HRMAS-NMR (600 MHz, CDCl₃): δ (ppm) = 0.9 (s, -CH₃), 1.3 (s, -CH₂), 3.6 (s, -OCH₂-, -CHO-, -CH₂N-), 3.7 (s, -OCH₃).

Cleavage of Lipid (6) from the resin: Resin bound lipid (6) was stirred in a 5% solution of trichloroacetic acid in dichloromethane. The resin was filtered and washed several times with CHCl₃/CH₃OH (4:1). The solutions were combined and evaporated under reduced pressure, and the product was purified by prep. HPLC. ¹H-NMR (400 MHz, CDCl₃): δ (ppm) = 0.8 (t, 6H, J = 6.5 Hz, -CH₃), 1.2 (br. S, 52 H, -CH₂-), 3.2 (s, 3H, N⁺-CH₃), 3.3 (m, 2 H, -NCH₂-), 3.4 (m, 2H, -CH₂O-), 3.6 (m, 1H, -CHO-)
MS (MALDI-TOF, DAB): m/z: 498,9 [M]⁺
Generally yields were in the region of 75-85%.

Exchange of counter ions (e.g. acetate, chloride, sulfate, methyl sulfate) was achieved by subsequent extraction of a solution of the respective lipid in CHCl₃/MeOH 4:1 with a saturated aqueous solution of sodium acetate (sodium acetate, sodium chloride, sodium sulfate and sodium methyl sulfate).

### II. Transfection studies

Cells and cell lines: Peripheral blood mononuclear leukocytes (PBL) were obtained from blood of healthy control persons isolated by Ficoll-Paque (Pharmacia Biotech AB, Uppsala, Sweden) density centrifugation. Dendritic cells (DC) were derived from highly purified circulating CD14(+) monocytes. The immunomagnetic purification of monocytes from PBL was performed according to manufacturer's instructions using CD14 Microbeads and MiniMACS high gradient magnetic separation columns (Miltenyi Biotech, Bergisch Gladbach, Germany). Isolated monocytes (5 H 10⁵/ml) were cultured with human recombinant GM-CSF (0.05µg/ml) and IL-4 (0.025µg/ml) (PromoCell, Heidelberg, Germany) in 6-well plates. Aliquots of the cells were reanalyzed by FACS with anti-CD14 mAbs (Pharmingen, San Diego, CA). After 5-6 days non-adherent cells were collected and purity of DCs was determined by FACS analysis. Flow cytometry was performed on a FACSort flow cytometer (Becton Dickinson, Mountain View, CA) using the CellQuest software for data processing as recently described ... using HLA class I (clone W6/32), HLA-DR (clone HB55) (Dr. G.DeLibero, Basel, Switzerland), and CD1a, CD3, CD11c, CD14, CD54, CD80, CD83 and CD86 (Pharmingen, San Diego, CA) specific mAbs and R-PE-goat anti-mouse Ig (Southern Biotechnology Associates, Inc., Birmingham, AL). Binding specificity was determined using IgG1 (MOPC-21), IgG2a (UPC-10), or IgG2b (MOPC-141) isotype control mAbs (SIGMA-ALDRICH Chemie, Buchs, Switzerland).
Mamma CA cell lines MDA-MB468 (ATCC HTB-132), and MCF7 (ATCC MTB-22) or canine normal kidney epithelial cell line MDCK (NBL-2) (ATCC CCL-34), and SV40 transformed African green monkey kidney cell line COS-7 (ATCC CRL-1651) were routinely passaged in DMEM and EMEM, (GIBCO BRL, Karlsruhe, Germany), respectively, supplemented with 1% L-Glutamin solution (100x), 1% Penicillin/Streptomycin solution (100x) (GIBCO BRL, Karlsruhe, Germany), and 10% FCS (BioWhittaker, Verviers, Belgium) at 37°C with 5% CO₂ in humified atmosphere.

Transfection Vector: Vector pEGFPLuc (BD Biosciences Clontech, Palo Alto, CA) is a commercial available reporter vector encoding a fusion protein of green fluorscent protein (GFP) and luciferase (Luc). All information are available at Clontech homepage (http://www.clontech.com/techinfo/vectors/vectorsE/pEGFPLuc.shtml).

Cell culture: The initial transfection screening was carried out using COS-7 cells. The cells were maintained in 96-well culture plates (Greiner, Frickenhausen, Germany) in 200 µl EMEM/10 % FCS/1 % Penicillin-streptomycin in a humidified atmosphere at 37 °C/5 % CO₂. 24 h prior to transfection, the cells were seeded into the microtiter plate at 5000-10,000 cells per well to reach a confluency of 50 % at the time of transfection.

Determination of the transfection properties: Determination of transfection behavior of the new lipids were performed using an automated assay system which was recently developed. In brief, the method consists of two parts, (i) the preparation of liposomes and formation of lipoplexes from the different lipids and (ii) cell transfection and determination of transfection properties.

Part 1 (Pretransfection assays): Cationic lipids in organic solvents were transferred into glass test tubes. The organic solvents were removed under a stream of nitrogen to create a thin lipid film on the surface of the glass tube. Buffer were added and the tubes were transported into a water bath sonicator where a dispersion of small cationic liposomes is formed. The liposomal dispersions are distributed to the wells of a 96 deep-well-plate and reporter plasmid DNA is added to allow formation (1 h) of the lipid/DNA-complexes (lipoplexes). For the subsequent quantification of transfection efficiency (part 2) a reporter plasmid carrying the firefly luciferase gene under the control of the CMV promoter is used to form lipoplexes.

Part 2 (Transfection assays): After lipoplex formation, the robot took the cell culture microtiter plates from an incubator and removed the lid from the plates. Immediately prior to transfection, 100 µl of the culture medium were removed and the lipoplexes were dispensed into the wells (triplicates). The lids were replaced and the MTP were returned to the incubator. After 4 hours the cells were automatically retrieved, the cell monolayers were carefully washed using the special drop mode a plate washer, fresh medium were added and the cells were incubated for a further 42 hours before harvesting. The MTP containing the cells were transported to the robot, the medium were removed, and the cells were washed using the drip mode of the microplate washer and lysed. Cell lysates were diluted and aliquots transferred to white microplates for the luciferase activity assay and transparent plates for the BCA protein assay. Assay specific standards and controls were added to the microplates, and luciferase activity and protein content of the lysates were measured. Transfection efficiencies were calculated by dividing luciferase activity by protein content. Dividing protein content of transfected cells by protein content of control non-transfected cells resulted in a relative measure of cytotoxicity.

Table 2 below summarizes the transfection efficiency of KL-14 in a mixture with Cholesterol (ratio: 0.7) or DOPE (dioleoylphospatidylethanolamine) (ratio: 1.0) toward the mamma carcinoma cell lines MDA-MB-468 and MCF-7, the polarized cell line MDCK-C7 and the primary dendritic cells. Transfection efficiencies of the KL-14 lipoplexes were compared to the transfection efficiency achieved with the standard transfection lipid DOTAP. Results were given in rlu/µg protein/ml (lower line) and, for easier comparison, standardized on the lipofection efficiency of DOTAP-lipoplexes (set as 1, upper line).

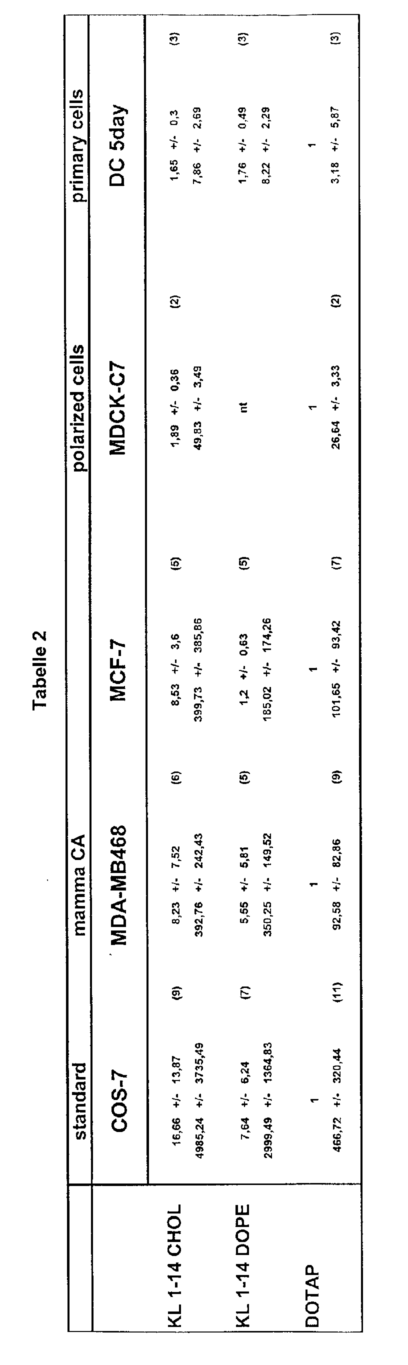

## Claims

1. A composition for transferring biologically active molecules into cells and/or tissues comprising
(a) a cationic lipid of formula (I) 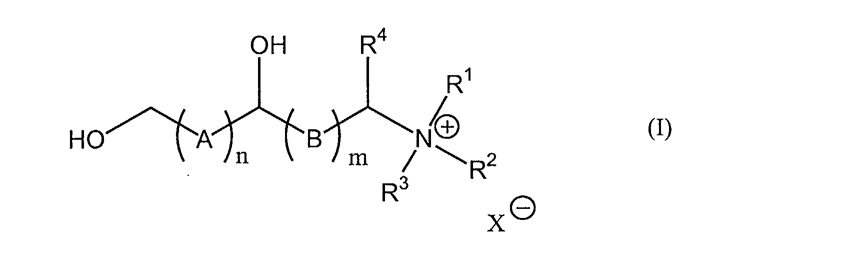 wherein
R¹, R² and R³ are the same or different and are selected from hydrogen and an alkyl group which may be substituted, provided that at least one of R¹, R² and R³ is an alkyl groups which may be substituted and that the total number of carbon atoms within R¹, R² and R³ is at least 18,
R⁴ is hydrogen or a lower alkyl residue having about 1 to 6 carbon atoms which may be substituted,
A and B are the same or different and are selected from-C(=O)-, -C(OH)R⁵- and -CR⁵R⁶- (wherein R⁵ and R⁶ are hydrogen or a lower alkyl residue having about 1 to 6 carbon atoms which may be substituted),
m and n are independently an integer of 0 to 3, and
X is a biologically and/or pharmaceutically acceptable counter ion, or derivatives of the lipid of formula (I); and
(b) at least one biologically active molecule.

2. The composition of claim 1, wherein in the cationic lipid (a)
(i) at least two of R¹, R² and R³ are alkyl groups which may be substituted; and/or
(ii) R¹ and R² are linear or branched chain, saturated or unsaturated alkyl residues having at least about 8, preferably 10 to 26 carbon atoms which may be substituted, more preferable R¹ and R² are linear alkyl residues having 10 to 22 carbon atoms, most preferable 12 to 20 carbon atoms; and/or
(iii) R³ is hydrogen or a saturated lower alkyl residue having about 1 to 6, preferably 1 to 3 carbon atoms which may be substituted, more preferable R³ is a methyl group or a hydroxy-lower-alkyl group; and/or
(iv) R⁴ is hydrogen, a lower alkyl group or a hydroxy-lower-alkyl group; and/or
(v) the substituents on the alkyl group and lower alkyl group are selected from hydroxy, oxy, halogen, lower alkyl, lower alkoxy, acyl, acyloxy, mercapto and lower alkylthio, and the number of substituents is 1 to 5, preferably 1 to 3;
(vi) X is selected from carboxylates, fluorinated carboxylates, sulfates, alkylsulfates, fluorinated alkylsulfates, arylsulfates, fluorinated arylsulfates and halides.

3. The composition of claim 1 or 2, wherein in the cationic lipd (a) n and m are 0, R³ is hydrogen, a methyl group or a hydroxyethyl group and R⁴ is hydrogen.

4. The composition of claim 3, wherein in the cationic lipid (a) R¹ and R² are saturated or unsaturated, linear C12, C14, C16, C18, C20 or C22 alkyl residues, R³ is a methyl group, and X is halide, acetate, methylsulfate or sulfate and preferably is chloride.

5. The composition of claim 4, wherein the configuration of C(2) of the cationic lipid (a) is racemic, or has either *R* or *S* configuration.

6. The composition according to any one of claims 1 to 5 wherein the biologically active molecule (b) is selected from the group consisting of nucleic acid constructs, DNA, RNA, synthetic polynucleotides, antisense polynucleotides, missense polynucleotides, nonsense polynucleotides, ribozymes, proteins, peptides, small molecular weight drugs, antibiotics and hormones.

7. The composition according to any one of claims 1 to 6 additionally comprising (c) helper compounds/lipids differing from the cationic lipid of formula (I) or a mixture of such helper lipids.

8. The composition of claim 7, wherein the helper compounds/lipids are selected from phosphatidylcholine, lyso-phospatidylcholine, phosphatidylethanolamine and lyso-phosphatidylethanolamine and the like (having saturated or unsatured carbon chains of variable length); sphingomyelin, ceramide and the like; membrane forming amphiphiles such as block polymers and the like; alkylester, -ether, -amides of sugars, dioles, polyoles, amines, amino acids, peptides and the like; cationic or lipophilized polymers such as DEAE-dextran, histones, polyethylene imine and the like; cholesterol; phosphatidylglyceroles, phosphatidylserines, phosphatidylinositoles and the like; helper agents modulating uptake of lipoplexes by macrophages and other cells such as gangliosides, phosphatidylpolyglycideroles, PEG-phosphatidylethanolamines, proteins (including IgG and Fc-fragments) and the like, preferably the helper lipid is dioleoylphospatidylethanolamine (DOPE) or cholesterol.

9. The composition as defined in claim 7 or 8, wherein the molar ratio of (c)/(a) is from 0.01 to 20, preferably 0.1 to 5, and more preferably is from 0.3 to 1.2 in the case of DOPE and from 0.3 to 1.0, most preferably from 0.5 to 0.7 in the case of cholesterol.

10. The composition as defined in any one of claims 1 to 9 which is a composition for gene therapy suitable for transferring a biologically active molecule into mammalian, preferably human cells.

11. Use of a cationic lipid of formula (I) as defined in claims 1 to 5
(i) as constituent of a transfection agent for transferring a biologically active molecule into cells and/or tissues *in vitro* or
(ii) for preparing an agent for transferring a biologically active molecule into cells and/or tissues *in vivo* including an agent for gene therapy.

12. The use of claim 11 wherein the agent further comprises a helper compound/lipid as defined in claims 7 to 9.

13. A cationic lipid of the formula (I) as defined in any one of claims 1 to 5, provided that a compound is excluded where m and n are 0, R¹ and R² are saturated linear and unsubstituted alkyl groups having 8 to 18 carbon atoms, R³ is methyl, R⁴ is hydrogen and X is a halide.

14. A process for the manufacture of the cationic lipid as defined in claims 1 to 5 which comprises
(a) reacting a compound of the formula (II) 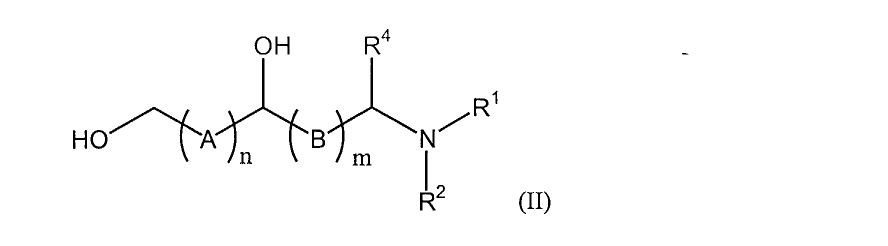 with a compound of the formula (III)
R³-Y (III)
wherein all variables are as defined in claims 1 to 5 and Y is a leaving group.

15. A method for transferring biologically active molecules into cells and/or into tissues or for gene therapy which method comprises contacting the cells or tissues, or the subject in need of the gene therapy with a composition comprising a cationic lipid of formula (I) as defined in claims 1 to 5.
